# EUROPEAN PATENT SPECIFICATION

(11) **EP 1 336 106 B1**
(45) Date of publication and mention of the grant of the patent: **17.11.2004**
(21) Application number: 01912100.3
(22) Date of filing: 15.02.2001
(51) Int. Cl.: G01N 33/569

(54) **DIAGNOSTIC METHOD FOR BACTERIAL VAGINOSIS BASED ON DETECTING ANTIBODIES AGAINST GVH TOXIN OF GARDNERELLA VAGINALIS**
DIAGNOSTISCHES VERFAHREN ZUR ERKENNUNG VON BAKTERIELLER VAGINOSE AUF BASIS DER BESTIMMUNG VON ANTI-GARDNERELLA-VAGINALIS-GVH-TOXIN ANTIKÖRPERN
PROCEDE SERVANT A DIAGNOSTIQUER UNE VAGINITE BACTERIENNE BASE SUR LA DETECTION D'ANTICORPS CONTRE LA TOXINE GVH DE GARDNERELLA VAGINALIS

(43) Date of publication of application: 20.08.2003
(73) Proprietor: UNIBIO S.r.l., 40125 Bologna (IT)
(72) Inventor: CAUCI, Sabina, I-34074 Monfalcone (IT)
(74) Representative: Siniscalco, Fabio
(86) International application number: PCT/IT2001/000068
(87) International publication number: WO 2002/065130

(56) References cited:
- CAUCI SABINA ET AL: "Specific immune response against Gardnerella vaginalis hemolysin in patients with bacterial vaginosis." AMERICAN JOURNAL OF OBSTETRICS AND GYNECOLOGY, vol. 175, no. 6, 1996, pages 1601-1605, XP001052898 ISSN: 0002-9378 cited in the application
- CAUCI SABINA ET AL: "Immunoglobulin A response against Gardnerella vaginalis hemolysin and sialidase activity in bacterial vaginosis." AMERICAN JOURNAL OF OBSTETRICS AND GYNECOLOGY, vol. 178, no. 3, March 1998 (1998-03), pages 511-515, XP001052896 ISSN: 0002-9378
- CAUCI SABINA ET AL: "Impairment of the mucosal immune system: IgA and IgM cleavage detected in vaginal washings of a subgroup of patients with bacterial vaginosis." JOURNAL OF INFECTIOUS DISEASES, vol. 178, no. 6, December 1998 (1998-12), pages 1698-1706, XP001052894 ISSN: 0022-1899
- CAUCI S ET AL: "Relationships among microbial factors activities and host mucosal immune response in bacterial vaginosis." INTERNATIONAL JOURNAL OF GYNECOLOGY & OBSTETRICS., vol. 67, no. Suppl. 1, November 1999 (1999-11), page S50 XP001052892 Second International Meeting on Bacterial Vaginitis.;Aspen, Colorado, USA; September 17-19, 1998 ISSN: 0020-7292
- CAUCI S ET AL: "Impairment of the mucosal immune response and microbial factors activities in bacterial vaginosis." IMMUNOLOGY LETTERS, vol. 69, no. 1, 15 June 1999 (1999-06-15), page 100 XP001052868 10th International Congress of Mucosal Imunology;Amsterdam, Netherlands; June 27-July 1, 1999 ISSN: 0165-2478

## Description

The current invention relates to a method for the evaluation of the risk of pathologies related with the colonization of women by the bacterium *Gardnerella vaginalis*.

In particular, this invention relates to a method for the qualitative and quantitative determination of the risk of pathologies of women colonized by the bacterium *G. vaginalis* such as, for example, low birth weight (LBW), preterm delivery (PTD), preterm rupture of membranes, intraamniotic infections, spontaneous abortion, endometritis, post-partum or post-gynecologic surgery infections, upper genital tract infections (PID) causing infertility, cervicitis, susceptibility to sexually transmitted diseases as viral infections from papillomavirus (HPV) and human immunodeficiency virus (HIV).

This invention is based on a method comprising the isolation of the hemolytic toxin Gvh produced by *G. vaginalis* in a culture medium and its use for the determination of the presence of the corresponding antibodies in a body fluid.

Low birth weight (LBW), primarily due to preterm birth (PTD), is the main risk factor for neonatal morbidity, mortality and long term infant neurological disorders.

Most cases of low birth weight have no recognized causal factors, making current prevention strategies ineffective. However, several investigations have shown that bacterial vaginosis (BV) is associated with preterm delivery (PTD), with an increased relative risk from 1.4 to 6.9 fold in respect to absence of BV, preterm delivery of low-weight infants, early and late miscarriage, and in general with maternal complications.

Moreover, recently it has been demonstrated that bacterial vaginosis is associated with increased risk of sexual acquisition of the viral infection by HIV and of vertical mother-to-child HIV transmission. Bacterial vaginosis prevalence is about 10% in non-pregnant women and about 15% in pregnant women in Western Europe, from 10 to 30% in US pregnant women, over 30% in colored African origin women, over 60% in women attending sexually transmitted disease clinics. Anyway, it is to be noted that only a small percentage of women with BV have adverse pregnancy outcomes or acquire HIV infection, among subjects at elevated risk of viral HIV exposure, so there is the requirement to have selective markers to individuate patients at elevated risk and that need to be therapeutically treated.

Bacterial vaginosis is caused by different bacterial species comprising *G. vaginalis*, *Bacteroides* spp., *Prevotella* spp. and *Mobiluncus* spp., *Ureaplasma urealyticum* and *Mycoplasma hominis.* The pathogenesis of bacterial vaginosis is still obscure and not always the presence of such pathologic condition is easily diagnosticable. In particular, it is not known what triggers the shift from the normal lactobacilli colonization to the altered vaginal flora, nor which degree of the vaginal microbial flora alteration is indicative of a real pathologic condition.

A crucial issue in bacterial vaginosis is the synergy between *G. vaginalis* and anaerobic bacteria.

*G. vaginalis* is found in high titers in over the 95% of bacterial vaginosis cases, whereas low *G. vaginalis* colonization titers are found also in women without bacterial vaginosis.

However, a strict correlation between *G. vaginalis* colonization and the condition of bacterial vaginosis has been demonstrated.

The only fully characterized virulence factor released by *G. vaginalis* is a β-hemolytic toxin (Gvh) having a molecular weight of about 59,000 Dalton.

It has been shown that about half of the non-pregnant women develop an immune response against Gvh eliciting IgA antibodies at the level of the vaginal mucosa. The absence of anti-Gvh IgA antibodies in non-pregnant women with BV is mainly due to the inactivation of IgA and this phenomenon is related to the presence of high levels of vaginal sialidase activity. Such inactivation derives from a structural impairment of IgA likely due to the hydrolytic action of an array of factors released by anaerobic bacteria.

Presently, it is not known which factors of the antimicrobial host defense are compromised or which microbial virulence factor(s) elicits the IgA cleavage in a subgroup of women with bacterial vaginosis.

It is not yet established if this condition is associated with increased risk of BV complications.

AMERICAN JOURNAL OF OBSTETRIC AND GYNECOLOGY, vol. 178, no. 3, March 1998 (1998-03), pages 511-515 describes the correlation between the IgA immune response to *Garnerella vaginalis* hemolysin and sialidase activity in vaginal fluids. Anti-hemolysin IgA levels were detected by ELISA with wells coated with purified *Garnerella vaginalis* toxin. Furthermore, sialidase activity can be a valuable diagnostic marker for predicting the severity of the disease. However, further studies are necessary to reveal as to whether sialidase activity can be a diagnostic marker.

JOURNAL OF INFECTIOUS DISEASE, vol. 178, no. 6, December 1998 (1998-12), pages 1698-1706 discloses a correlation between IgA degradation and the absence of immune response to *Garnerella vaginalis* cytolysin. The extent of IgA degradation correlates also with sialidase activity level. Patients with bacterial vaginosis having high sialidase activity and extensive IgA degradation in their secretions could incur more dangerous infections and adverse pregnancy outcomes. IgG was shown to be intact in all women. Prolidase activity was also measured. Furthermore, the pH of vaginal secretions was detected. However, again further studies will be needed to verify whether patients are at risk of complications and so identify women who really need treatment.

Such an uncertain definition makes clinicians to face the problem whether or not administrate a pharmacological therapy to women with bacterial vaginosis. It is well known that two different drugs are used to treat bacterial vaginosis: metronidazole and clindamycin. Both these drugs show a main withdraw consisting in relevant adverse side effects, in particular metronidazole can give gastrointestinal symptoms, and is considered as a teratogen agent, so it is not recommended during pregnancy, on the other hand clindamycin can cause diarrhea and pseudomembranous colitis which may be lethal. Moreover both such antibiotics can cause yeast vaginitis.

For the above described reasons, it is highly desirable the development of a method to determine the risk of pathologic complications in women colonized by the *G. vaginalis* bacterium, as, for example, low birth weight or preterm birth, spontaneous abortion, endometritis, acquisition of HIV infection, of HPV infection (which causes cervical intraepithelial neoplasia), upper genital tract infections (PID), post-partum and post-gynecologic surgery infections.

The present invention provides a reliable method to solve the problem of the risk determination of the above-described complications in a way to furnish the physician a valuable tool to decide whether or not give a pharmacological therapy.

Such a problem is solved by a method for determination of risk of pathologies as expressed in the main enclosed claim.

Further features and advantages of the present invention method will be highlighted by the subsequent description of preferred embodiments, given at indicative and not limitative title.

For the setting up of the method of the current invention clinical cases have been studied and compared with controls.

In particular, 116 women who had low birth weight babies (LBW), 86 women who had a preterm delivery (PTD), and 417 women who had normal birth weight babies at term of gestation (NTD) were examined.

The presence of *G. vaginalis* and the levels of anti-Gvh IgA were evaluated in the vaginal fluids of all these women.

In particular, the vaginal fluid samples were obtained from women in the first or in the second trimester of gestational age, preferably in the period ranging from the 7 to the 24 full weeks' gestation.

These groups of women have been further subdivided on the basis of the presence, in the vaginal fluids samples, of *G. vaginalis* alone, *G. vaginalis* and a pH value equal or higher than 4.7, *G. vaginalis* and the pathologic condition bacterial vaginosis (BV), *G. vaginalis* in association with anaerobic bacteria, *G. vaginalis* in association with the "*Bacteroides* group", and *G. vaginalis* in association with anaerobic bacteria different from *Bacteroides* that we called "not specific".

In the current description with the term "*Bacteroides* group" we indicate a subgroup of anaerobic bacteria comprising the isolates of *Bacteroides* spp., *Prevotella* spp., *Porphyromonas* spp. and strains of *Bacteroides fragilis* group. With the term "not specific" anaerobic bacteria we instead call a subgroup of anaerobic bacteria comprising all other anaerobes as, for example, species of *Bifidobacterium*, *Peptostreptococcus, Propionibacterium, Clostridium* and *Veillonella*.

In the following Table 1, the association between anti-Gvh IgA response and low birth weight (LBW) or preterm birth (PTD) is shown.

**TABLE I**

| | Positive %, **OR** | | | | |
|---|---|---|---|---|---|
| **Vaginal colonization** **and anti-Gvh IgA response** | **NTD** **n=417** | **LBW** **n=116** | | **PTD** **n=86** | |
| ***G. vaginalis*** | 36.2 | 42.2 | **1.3** | 36.0 | **1.0** |
| **no or low anti-Gvh IgA** | 29.5 | 39.6 | **1.6** | 33.7 | **1.2** |
| **high anti-Gvh IgA** | 6.7 | 2.6 | **0.4** | 2.3 | **0.3** |
| ***G. vaginalis* and pH ≥ 4.7** | 21.8 | 31.9 | **1.6** | 24.4 | **1.2** |
| **no or low anti-Gvh IgA** | 18.2 | 31.0 | **1.8** | 24.4 | **1.4** |
| **high anti-Gvh IgA** | 3.6 | 0.9 | **0.3** | 0.0 | **-** |
| ***G. vaginalis* and BV** | 12.9 | 20.7 | **1.7** | 10.5 | **0.8** |
| **no or low anti-Gvh IgA** | 11.0 | 20.7 | **2.1** | 10.5 | **1.0** |
| **high anti-Gvh IgA** | 1.9 | 0.0 | **-** | 0.0 | **-** |
| ***G. vaginalis* and anaerobes** | 9.8 | 19.0 | **2.1** | 11.6 | **1.2** |
| **no or low anti-Gvh IgA** | 7.4 | 19.0 | **2.9** | 11.6 | **1.7** |
| **high anti-Gvh IgA** | 2.4 | 0.0 | **-** | 0.0 | **-** |
| ***G. vaginalis* and *Bacteroides* group** | 5.0 | 6.9 | **1.4** | 4.7 | **0.9** |
| **no or low anti-Gvh IgA** | 3.6 | 6.9 | **2.0** | 4.7 | **1.3** |
| **high anti-Gvh IgA** | 1.4 | 0.0 | - | 0.0 | ***-*** |
| ***G. vaginalis* and not-spec. anaerobes** | 5.0 | 15.5 | **3.5** | 8.1 | **1.7** |
| **no or low anti-Gvh IgA** | 3.8 | 15.5 | **4.4** | 8.1 | **2.2** |
| **high anti-Gvh IgA** | 1.2 | 0.0 | **-** | 0.0 | **-** |

In the above shown Table 1, the figures in regular font indicate the percentages of women of each of the previous defined groups. Moreover, for each said group the percentages of women with no or low levels of IgA, and high levels of IgA against the toxin Gvh are reported.

In bold font, instead, it is reported the ratio (odds ratio, OR) between the percentage of women who had LBW or PTD and the percentage of NTD women. This value was calculated and corrected with a standard factor by means of the SPSS computer statistical program.

From the analysis of the clinical data reported in Table 1 it is possible to relate the relative risk of LBW or PTD with the level of IgA, and, in a further step, with the bacterial flora present in the body fluid of the patient.

As a matter of fact, the relative risk of low birth weight (LBW) or of preterm birth (PTD) is evaluated by the OR ratio.

If this ratio is lower than 1 it means that the woman has no or low risk of complications. A ratio ranging from 1 to 2 is associated with a medium risk of complications. If this ratio is comprised between 2 and 4 it means that the woman has a high risk of complications. If this ratio is higher than 4 it means that the woman has a very high risk of low birth weight or preterm birth.

In particular, and for example, a medium risk corresponding to an OR value equal 2 means that the patient has a twofold risk of complications compared to a normal woman, that is, in other words, a 100% higher.

Preferably, this result can be point out with a score: - meaning a low or no risk, + meaning a medium risk, ++ meaning a high risk, and +++ meaning a very high risk.

The above shown Table 1 highlights that the risk of low birth weight or preterm birth, independently from the presence of other bacteria different from *G. vaginalis* and independently from the presence of the BV pathologic condition, is essentially absent or very low for women having high levels of anti-Gvh IgA in the vaginal fluid sample.

As a consequence the presence of high levels of anti-Gvh IgA is an indication of a protective effect against these risks. This permits to isolate patients with altered bacterial flora that do not need a therapeutic treatment. Such a finding is of major relevance in the case of pregnant women and in patients allergic to antibiotics.

On the basis of these studies and these results, a method to determine the pathologic risk of women colonized by the *G. vaginalis* bacterium has been set up.

In agreement with the current invention, the method for the determination of the risk of pathologies of women colonized by the *G. vaginalis* bacterium (and/or with bacterial vaginosis, BV) comprises the following steps in the order:
a) determination of levels of IgA and/or IgG and/or IgM antibodies against the Gvh toxin produced by *G. vaginalis* in a body fluid sample;
b) comparison of such levels of IgA and/or IgG and/or IgM with predetermined levels of IgA and/or IgG and/or IgM, respectively;
c) determination of the risk factor.

This method comprises a first step of evaluation of biochemical data and a second step of comparison of such data. The first step consists in the determination of the levels of antibodies against the hemolytic toxin Gvh produced by the bacterium *G. vaginalis* and secreted at the level of the vaginal mucosa.

The determination of the levels of anti-Gvh IgA is performed by any suitable immune enzymatic assay as, for example, ELISA, RIA, Western-blot analysis, dot blot, spot test, dipstick, immunicard, Quick Card.

Preferably, in the realization of the current invention an ELISA test was used, as described in Cauci et al., Am J Obstet Gynecol 1996; 175: 1601-5.

This test consists, in general, in the wells' incubation with a prefixed quantity of purified toxin dissolved in a suitable buffer at room temperature. Wells are then properly washed and re-incubated with a substance able to block the free binding sites left after the coating with the toxin. Later on, a fixed volume of a properly processed sample of vaginal fluid is added and incubated at 37°C. Wells are then washed again and incubated with suitable anti-human IgA antibodies. Finally, the quantitative determination is obtained by the measurement of a color development. Results are reported as milli-optical density units (mOD).

The color development was obtained by means of the para-nitrophenylphosphate substrate that releases para-nitrophenol after the hydrolysis done by the alkaline phosphatase conjugated to the anti-human IgA antibody.

The second step comprises the determination of the value to take into account to evaluate the risk.

In particular, it has been determined a cutoff value of 390 mOD units from the mean value plus one standard deviation calculated in a healthy reference control group of women population. Below this cutoff it is assumed that no specific immune response anti-Gvh IgA has been raised, whereas values over or equal 390 mOD and below 780 mOD (twofold the first cutoff) are considered as a low immune response. Finally, values over or equal 780 mOD are considered as a high immune response.

So it is evident that if a vaginal fluid sample shows IgA levels over 780 mOD it means that the risk of pregnancy complications is low or absent regardless, as previously illustrated, from the presence of determined bacteria or of the pathologic condition bacterial vaginosis.

Alternatively, it is possible to use other substrates to determine the cutoff value as, for example, 2-nitrophenylphosphate, or the combination BCIP/NBT (5-bromo-4-chloro-3-indolylphosphate / nitro blue tetrazolium) which develops a blue/violet hydrolysis product, or 4-methylumbelliferyl phosphate which gives a blue fluorescent hydrolysis product.

Moreover, alternatively, the enzyme conjugated to the anti-human Ig antibody can be different from the alkaline phosphatase, as for example peroxidase or beta-galactosidase and the enzymatic activity would be evaluated with a suitable substrate.

Finally, the anti-human Ig antibody could be conjugated with biotin which could be detected by avidin conjugated to alkaline phosphatase or peroxidase or beta-galactosidase.

In all these alternative procedures the cutoff will be evaluated by calculation of the mean value plus a standard deviation obtained in a healthy control population, the value defining a high antibody response will be a value twofold the first cutoff.

From all what described till now, it is evident that the method of this invention allows to determine in a qualitative manner the risk of pathologies from the identification of low or no levels of anti-Gvh IgA.

Profitably, furthermore, the method of this invention permits to have a quantitative determination of such risk. This determination relies on a precise risk evaluation taking into account the samples in which low or no anti-Gvh IgA levels have been found.

According to the current invention, the method can comprise a step of pH determination in the vaginal fluid. pH is calculated by means of well known methods as, for example, pH meter or pH revealing paper strip with a turning range between 4 and 7.

The method of this invention can so include after the phase b), the sample pH determination and the subsequent comparison of the obtained value with a prefixed pH.

As it is shown in Table 1, the OR value for a pH value equal or over 4.7 and low anti-Gvh IgA levels is included in the range from 1 to 2, both for LBW and PTD.

As a consequence, when the IgA levels are below 780 mOD and the pH value in the vaginal fluid samples is equal or over 4.7, according the method of this invention, the evaluated risk is medium for PTD and for LBW.

According to a further embodiment of the invention, the method can be performed using vaginal fluid samples of women with diagnosis of bacterial vaginosis (BV).

As shown in Table 1, the OR value, still corresponding to the absence or low levels of anti-Gvh IgA, is in the range from 2 to 4 (2.1 ) for LBW, and in the range from 1 to 2 (1.0) for PTD.

It results that the method of the invention can comprise after the step b), the determination of samples from women with BV.

Consequently, according to the method of this invention if the vaginal fluid sample derives from women having BV and if low or no IgA levels have been found, it turns out that the risk of complications is medium for PTD and high for LBW.

According to a further embodiment of the current invention, the method can comprise a step of bacterial flora determination in the vaginal fluid samples of pregnant women by means of microbiological culture performed using routine methods well known in the field.

The evaluation of risk is performed thorough the presence of anaerobic bacteria in general, or, more in detail, of the *Bacteroides* group or the not specific anaerobic bacteria group.

The different groups of bacteria are isolated with well-known procedures (Thorsen et al., Am J Obstet Gynecol 1998; 178: 580-7 - Lautrop et al., FADL's forlag, Copenhagen, Denmark, 1979).

In particular, strains of anaerobic bacteria are identified and isolated, and then they are clustered in the *Bacteroides* group and in the group of not specific anaerobic bacteria, as above described.

As shown in Table 1, considering vaginal fluid samples having low or no levels of anti-Gvh IgA and retrieved from women colonized by anaerobic bacteria, or by the *Bacteroides* group bacteria, the OR for preterm birth (PTD) is in the range between 1 and 2 (1.7 and 1.3, respectively), whereas the OR value for low birth weight (LBW) is in the range from 2 to 4 (2.0 and 2.9, respectively).

On the other hand, if in the vaginal fluid samples not specific anaerobic bacteria have been found, the OR for low birth weight (LBW) is over 4 (4.4), whereas the OR value for preterm birth (PTD) is in the range from 2 to 4 (2.2).

The method by the terms of this invention can so include after the phase b), the bacterial flora determination in said sample.

From what just described it comes out that:
- if anaerobic bacteria or *Bacteroides* group bacteria have been identified in the analyzed sample the risk for LBW is high, whereas the risk for PTD is medium;
- if not specific anaerobic bacteria have been identified in the analyzed sample the risk for LBW is very high, whereas the risk for PTD is high;
So it can be noted that in each of the considered case the evaluation of the anti-Gvh IgA response permits to do a more precise evaluation of risk as it allows to individuate women at very high risk and women not at risk very carefully.

According to a further embodiment of the invention, the method provides a further step consisting in the sialidase and prolidase activity determination in these samples.
Sialidases are enzymes produced by bacteria and are involved in the pathogenesis of several diseases. These enzymes provoke the hydrolysis of sialic acid from various proteins including IgA, altering the immune response.

Prolidases are proteolytic enzymes produced by bacteria to favor the cell infiltration. These enzymes are able to activate cytokines and other immune mediators.

The sialidase activity was determined by incubation of 50 µl of the vaginal fluid sample with 50 µl of a substrate at pH 5.0, with a procedure described by Cauci et al., Am J Obstet Gynecol 1998; 178: 511-5. Specific activity was expressed as nanomoles of methoxyphenol produced from conversion of the substrate and calculated by comparison with a standard curve of pure methoxyphenol. The sialidase activity levels were defined as: no activity for values below 0.19 nmol of methoxyphenol, +1 cutoff for values equal or above 0.19 nmol of methoxyphenol; +2 cutoff for values equal or above 0.38 nmol of methoxyphenol; +3 cutoff for values equal or above 2.50 nmol of methoxyphenol, and +4 cutoff for values equal or above 5.0 nmol of methoxyphenol.

Prolidase activity was determined as described by Cauci et al., J Infect Dis 1998; 178:1698-706. The prolidase activity levels were defined as: no activity for values below 22 mOD, +1 cutoff for values equal or above 22 mOD; +2 cutoff for values equal or above 44 mOD; +3 cutoff for values equal or above 1000 mOD and +4 cutoff for values equal or above 2000 mOD.

All the +1 cutoffs of enzymatic activity were based on the mean value plus one standard deviation measured in a healthy control population. The +2 cutoff was obtained by doubling the +1 cutoff.

In particular, according to the method of the invention for the determination of the risk of pathologies in women colonized by *G. vaginalis*, the combination of the no or low anti-Gvh IgA response with high values of sialidase or prolidase activity (+4) was associated with low birth weight or preterm birth in women with altered vaginal flora.

These groups of women were divided for the presence of bacterial vaginosis or not specific anaerobic bacteria.

The following Table 2 shows data analyzed as percentage of women which had low birth weight babies (LBW), preterm delivery (PTD) or normal term delivery (NTD), whereas data in bold show the ratio (OR) between per percentage of women with LBW, PTD and the percentage of NTD women, respectively.

This value was calculated and corrected by a standard factor by the SPSS computer statistic program.

The OR ratio, still in this picture, indicates the risk of LBW or PTD.

**TABLE 2**

| | Positive % **OR** | | | | |
|---|---|---|---|---|---|
| **Vaginal colonization** | **NTD** | **LBW** | | **PTD** | |
| **Bacterial vaginosis (BV)** | | | | | |
| **no or low anti-Gvh IgA** | | | | | |
| sialidase 4+ | 7.7 | 16.7 | **2.4** | 20.0 | **3.0** |
| prolidase 4+ | 1.9 | 12.5 | 7.3 | 10.0 | 5.7 |

| **pH ≥ 4.7** | | | | | |
|---|---|---|---|---|---|
| **no or low anti-Gvh IgA** | | | | | |
| sialidase 4+ | 2.2 | 9.3 | **4.5** | 7.1 | **3.5** |
| prolidase4+ | 2.2 | 9.3 | **4.5** | 7.1 | **3.5** |

| ***G. vaginalis*** | | | | | |
|---|---|---|---|---|---|
| **no or low anti-Gvh IgA** | | | | | |
| sialidase 4+ | 3.3 | 8.7 | **2.8** | 6.7 | **2.2** |
| prolidase 4+ | 2.4 | 10.9 | **4.9** | 10.3 | **4.6** |

| **Not specific anaerobic bacteria** | | | | | |
|---|---|---|---|---|---|
| **no or low anti-Gvh IgA** | | | | | |
| sialidase 4+ | 0.0 | 22.2 | ↑↑↑ | 28.6 | ↑↑↑ |
| prolidase 4+ | 4.8 | 16.7 | **4.1** | **28.6** | **8.0** |

The same approach utilized for estimating the values in Table 1 can be employed for the values in Table 2.

As a consequence, high levels of sialidase activity and low or no anti-Gvh IgA levels in vaginal fluid samples of women with bacterial vaginosis or in vaginal fluid samples in which *G. vaginalis* was found are indicative of a high risk of LBW, and PTD. Whereas, for the same levels of sialidase activity, in the vaginal fluid samples in which a pH value equal or higher than 4.7 was measured, the OR value for LBW is higher than 4 (4.5), and the OR value for PTD is comprised between 2 and 4 (3.5), so the risk for LBW is very high and the risk for PTD is high.

In Table 2 the arbitrary symbol (↑↑↑) was used in correspondence to the OR value associated with not specific anaerobes and no or low anti-Gvh IgA and very high sialidase activity, because the percentage of NTD women of this kind is zero and the ratio OR would come infinite.

So it results that the finding of the association not specific anaerobes and no or low anti-Gvh IgA and very high sialidase activity, is a 100% specific test for LBW and PTD.

No or low anti-Gvh IgA response and very high levels of prolidase activity in women with BV or colonized by *G. vaginalis* are associated with a very high risk for LBW and PTD.

For women having a vaginal pH equal or above 4.7 and very high levels of prolidase activity, the OR value for LBW is higher than 4 (4.5), whereas the OR value for PTD is in the range from 2 to 4 (3.5), so the risk for LBW is very high and the risk for PTD is high.

Finally, the finding of not specific anaerobic bacteria and no or low levels of anti-Gvh IgA and very high levels of prolidase activity corresponds to a very high risk for LBW and PTD.

In general, low anti-Gvh IgA levels combined with a very high prolidase activity are associated with very high risk values.

Following these results, the method to determine the risk of complications additionally comprises after the step b) the determination of the sialidase and/or prolidase activity in the said sample.

According to a further embodiment of the current invention, levels of IgG or IgM were analyzed in relation to LBW or PTD and the corresponding values are reported in the Tables from 3 to 6 described later on.

The IgG values were calculated in the same way as were calculated the IgA values, the only difference is that an anti-human IgG antibody was employed to perform the ELISA test.

The cutoff value to define the absence of IgG antibody response was 370 mOD (mean value plus one standard deviation evaluated in the healthy control population). A value comprised between 370 and 740 mOD indicates the raising of a low IgG antibody response. A value over 740 mOD (double of the first cutoff calculated from the mean value plus one standard deviation in the healthy control population) corresponds to a high IgG antibody response.

It is to notice that the results are very similar to those obtained measuring IgA, so that the same evaluations and considerations previously reported can be done.

IgM values were calculated after a treatment of the sample before the ELISA test. By this treatment the IgG are eliminated from the sample by means of "goat anti-human absorbent" (ProSorb G), that is a resin coupled with anti-human IgG antibodies well known in the field. Then the sample is concentrated from 5 to 10 folds with microcentrifuge filter units. This way to concentrate is performed by centrifugation employing a polysulfone membrane, which concentrates proteins with a molecular weight over 100,000 Dalton.

Finally, the ELISA test is performed as above described but using an anti-human IgM antibody.

The cutoff value for the absence of IgM antibody response was 300 mOD (mean value plus one standard deviation calculated for the healthy control population). A value comprised from 300 mOD and 600 mOD means that a low IgM antibody response was raised. Finally, the value 600 mOD (twofold the mean value plus one standard deviation calculated for the healthy control population) defines a high IgM response.

The evaluation of the method is parallel to the method used for IgA evaluation, so the following Tables will not be commented.

**TABLE 3**

| | Positive % **OR** | | | | |
|---|---|---|---|---|---|
| **Vaginal colonization** **and anti-Gvh IgG response** | **NTD** **n=417** | **LBW** **n=116** | | **PTD** **n=86** | |
| ***G. vaginalis*** | 36.2 | 42.2 | **1.2** | 36.0 | **1.0** |
| **no or low anti-Gvh IgG** | 27.8 | 39.6 | **1.4** | 33.7 | **1.2** |
| **high anti-Gvh IgG** | 8.4 | 2.6 | **0.3** | 2.3 | **0.2** |
| ***G. vaginalis* and pH ≥ 4.7** | 22.8 | 31.9 | **1.4** | 24.4 | **1.2** |
| **no or low anti-Gvh IgG** | 19.0 | 31.0 | **1.7** | 23.2 | **1.3** |
| **high anti-Gvh IgG** | 3.8 | 0.9 | **0.3** | 1.2 | **0.3** |
| ***G. vaginalis* and BV** | 12.9 | 20.7 | **1.7** | 10.5 | **0.8** |
| **no or low anti-Gvh IgG** | 10.8 | 20.7 | **1.9** | 9.3 | **1.0** |
| **high anti-Gvh IgG** | 2.2 | 0.0 | **-** | 1.2 | **0.6** |
| ***G. vaginalis* and anaerobes** | 9.8 | 19.0 | **2.1** | 11.6 | **1.2** |
| **no or low anti-Gvh IgG** | 7.2 | 19.0 | **2.7** | 10.5 | **1.6** |
| **high anti-Gvh IgG** | 2.6 | 0.0 | **-** | 1.2 | **0.5** |
| ***G. vaginalis* and *Bacteroides* group** | 5.0 | 7.0 | **1.4** | 4.7 | **0.9** |
| **no or low anti-Gvh IgG** | 3.6 | 7.0 | **2.0** | 3.5 | **1.1** |
| **high anti-Gvh IgG** | 1.4 | 0.0 | - | 1.2 | **0.9** |
| ***G. vaginalis* and non-spec. anaerobes** | 5.0 | 15.5 | **3.5** | 8.1 | **1.7** |
| **no or low anti-Gvh IgG** | 3.6 | 15.5 | **4.5** | 8.1 | **2.2** |
| **high anti-Gvh IgG** | 1.4 | 0.0 | - | 0.0 | - |

**TABLE 4**

| | Positive % **OR** | | | | |
|---|---|---|---|---|---|
| **Vaginal colonization** | **NTD** | **LBW** | | **PTD** | |
| **Bacterial vaginosis (BV)** | | | | | |
| **no or low anti-Gvh IgG** | | | | | |
| sialidase 4+ | 7.7 | 16.7 | **2.4** | 20.0 | **3.0** |
| prolidase 4+ | 3.8 | 12.5 | **4.0** | 10.0 | **2.7** |

| **pH** ≥ **4.7** | | | | | |
|---|---|---|---|---|---|
| **no or low anti-Gvh IgG** | | | | | |
| sialidase 4+ | 3.3 | 9.3 | **2.9** | 7.1 | **2.3** |
| prolidase 4+ | 2.2 | 9.3 | **4.5** | 7.1 | **3.5** |

| ***G. vaginalis*** | | | | | |
|---|---|---|---|---|---|
| **no or low anti-Gvh IgG** | | | | | |
| sialidase4+ | 4.1 | 8.7 | **2.3** | 6.7 | **1.7** |
| prolidase 4+ | 3.3 | 13.0 | **4.0** | 10.3 | **3.3** |

| **Not-specific anaerobic bacteria** | | | | | |
|---|---|---|---|---|---|
| **no or low anti-Gvh IgG** | | | | | |
| sialidase 4+ | 0 | 27.8 | ↑↑↑ | 28.6 | ↑↑↑ |
| prolidase 4+ | 4.8 | 16.7 | **4.0** | 28.6 | **8.0** |

**TABLE 5**

| | Positive % **OR** | | | | |
|---|---|---|---|---|---|
| **Vaginal colonization** **and anti-Gvh IgM response** | **NTD** **n=417** | **LBW** **n=116** | | **PTD** **n=86** | |
| ***G. vaginalis*** | 36.2 | 42.2 | **1.2** | 36.0 | **1.0** |
| **no or low anti-Gvh IgM** | 31.2 | 39.6 | **1.3** | 33.7 | **1.1** |
| **high anti-Gvh IgM** | 5.0 | 2.6 | **0.5** | 2.3 | **0.5** |
| ***G. vaginalis* and pH ≥ 4.7** | 21.8 | 30.2 | **1.5** | 23.2 | **1.1** |
| **no or low anti-Gvh IgM** | 18.2 | 29.3 | **1.7** | 23.2 | **1.3** |
| **high anti-Gvh IgM** | 3.6 | 0.9 | **0.3** | 0.0 | - |
| ***G. vaginalis* and BV** | 12.9 | 20.7 | **1.7** | 10.5 | **0.8** |
| **no or low anti-Gvh IgM** | 11.3 | 20.7 | **1.9** | 10.5 | **1.0** |
| **high anti-Gvh IgM** | 1.7 | 0.0 | - | 0.0 | - |
| ***G. vaginalis* and anaerobes** | 9.8 | 19.0 | **2.1** | 11.6 | **1.2** |
| **no or low anti-Gvh IgM** | 7.4 | 19.0 | **2.9** | 11.6 | **1.7** |
| **high anti-Gvh IgM** | 2.4 | 0.0 | **-** | 0.0 | **-** |
| ***G. vaginalis* and *Bacteroides* group** | 5.0 | 6.9 | **1.4** | 4.7 | **0.9** |
| **no or low anti-Gvh IgM** | 3.6 | 6.9 | **2.0** | 4.7 | **1.3** |
| **high anti-Gvh IgM** | 1.9 | 0.0 | **-** | 0.0 | **-** |
| ***G. vaginalis* and non-spec. anaerobes** | 5.0 | 15.5 | **3.5** | 8.1 | **1.7** |
| **no or low anti-Gvh IgM** | 4.6 | 15.5 | **3.7** | 8.1 | **1.9** |
| **high anti-Gvh IgM** | 0.5 | 0.0 | **-** | 0.0 | **-** |

**TABLE 6**

| | % Positivi **OR** | | | | |
|---|---|---|---|---|---|
| **Vaginal colonization** | **NTD** | **LBW** | | **PTD** | |
| **Bacterial vaginosis (BV)** | | | | | |
| **no or low anti-Gvh IgM** | | | | | |
| sialidase 4+ | 7.7 | 16.7 | **2.4** | 20.0 | **3.0** |
| prolidase 4+ | 1.9 | 12.5 | **7.3** | 10.0 | **5.7** |

| **pH** ≥ **4.7** | | | | | |
|---|---|---|---|---|---|
| **no or low anti-Gvh IgM** | | | | | |
| sialidase 4+ | 3.3 | 9.3 | **2.9** | 7.1 | **2.3** |
| prolidase 4+ | 2.2 | 6.9 | **3.4** | 7.1 | **3.5** |

| ***G. vaginalis*** | | | | | |
|---|---|---|---|---|---|
| **no or low anti-Gvh IgM** | | | | | |
| sialidase 4+ | 3.3 | 8.7 | **2.8** | 10.3 | **3.3** |
| prolidase 4+ | 2.4 | 10.9 | **4.9** | 10.3 | **4.6** |

| **Not-specific anaerobic bacteria** | | | | | |
|---|---|---|---|---|---|
| **no or low anti-Gvh IgM** | | | | | |
| sialidase 4+ | 0.0 | 22.2 | ↑↑↑ | 28.6 | ↑↑↑ |
| prolidase 4+ | 4.8 | 16.7 | **4.0** | 28.6 | **8.0** |

As mentioned in the introductive part of the current description, the method according to the invention profitably permits to determine the risk of pathologies as acquisition of the HIV viral infection, HPV viral infection, upper genital tract infections (PID), post-partum or post-gynecologic surgery infections, spontaneous abortion, endometritis.

In the following part, an example regarding a study of acquisition of the HIV viral infection will be described.

For setting the method, a group of 50 women colonized by *G. vaginalis* and at high risk of sexual acquisition of HIV infection was initially enrolled. Three years later among these women a subgroup of 42 women were still HN seronegative (controls) and a subgroup of 8 women were HIV seropositive.

These groups were subdivided according to the presence in the body fluid samples of *G. vaginalis* alone or *G. vaginalis* and a pH equal or higher than 4.7, or *G. vaginalis* and diagnosis of bacterial vaginosis (BV).

Table 7 shows the association of the anti-Gvh IgA response with the subgroup of the 42 HIV seronegative women (controls) or the 8 women which became HIV seropositive.

**TABLE 7**

| | Positive % **OR** | | |
|---|---|---|---|
| **Vaginal colonization** **and anti-Gvh IgA response** | **HIV seronegative** **n=42** | **HIV seropositive** **n=8** | |
| ***G. vaginalis*** | 100 | 100 | |
| **no or low anti-Gvh IgA** | 71.4 | 100 | **1.7** |
| **high anti-Gvh IgA** | 28.6 | 0.0 | |
| ***G. vaginalis* and pH ≥ 4.7** | 81.0 | 87.5 | **1.2** |
| **no or low anti-Gvh IgA** | 54.8 | 87.5 | **1.8** |
| **high anti-Gvh IgA** | 26.2 | 0.0 - | |
| ***G. vaginalis* and BV** | 71.4 | 87.5 | **1.4** |
| **no or low anti-Gvh IgA** | 50.2 | 87.5 | **2.1** |
| **high anti-Gvh IgA** | 19.0 | 0.0 - | |

The reported values have the same meaning described in the Tables concerning the LBW and PTD, for this reason they will not here described in details.

Anyway, Table 7 highlights that once again high levels of anti-Gvh IgA are indicative of a protective effect in respect to the risk of HIV acquisition.

Moreover, the OR ratio in the case of pH equal or higher than 4.7 and no or low anti-Gvh IgA response in the body fluid is in the range from 1 to 2 (1.8). This means that the risk of HIV acquisition is medium.

The OR ratio in women with BV and with no or low anti-Gvh IgA in the body fluid is in the range from 2 to 4 (2.1). This means that the risk of HIV acquisition is high.

As a consequence, the method according to the invention provides the same subsequent steps described for the determination of the risk for LBW and PTD.

According to an embodiment of the invention, the method for the determination of the risk of HIV infection can include a step of sialidase and/or prolidase activity determination as described for LBW or PTD.

The following Table 8 describes values corresponding to high sialidase or prolidase activity levels measured in fluid samples from women with bacterial vaginosis, or with pH ≥ 4.7 or colonized simply by *G. vaginalis*. As reported, in all the considered cases no or low IgA were detected.

**TABLE 8**

| | % Positivi **OR** | | |
|---|---|---|---|
| **Vaginal colonization** | **HIV seronegative** **(n. 42)** | **HIV seropositive** **(n. 8)** | |
| **Bacterial vaginosis (BV)** | | | |
| **no or low anti-Gvh IgA** | | | |
| sialidase 4+ | 13.3 | 42.9 | **3.5** |
| prolidase 4+ | 13.3 | 57.1 | **4.5** |

| **pH ≥ 4.7** | | | |
|---|---|---|---|
| **no or low anti-Gvh IgA** | | | |
| sialidase 4+ | 11.8 | 42.9 | **3.8** |
| prolidase 4+ | 14.7 | 57.1 | **4.2** |

| ***G. vaginalis*** | | | |
|---|---|---|---|
| **no or low anti-Gvh IgA** | | | |
| sialidase 4+ | 9.5 | 37.5 | **4.2** |
| prolidase 4+ | 11.9 | 50.0 | **4.4** |

In particular, for high levels of sialidase activity the OR is in the range from 2 to 4 in women with BV or women with a vaginal pH ≥ 4.7, whereas the OR is higher than 4 for women colonized by *G. vaginalis*.

Moreover, for all subsets shown in Table 8 high levels of prolidase activity were associated with OR always higher than 4.

From data reported in the Table 8, it is clear that if the levels of anti-Gvh IgA are low or absent and if the sialidase activity is higher than 5.0 nmol of methoxyphenol (high values, +4 cutoff) the risk is high; if the levels of anti-Gvh IgA are low or absent and if the prolidase activity is higher than 2000 mOD (high values, +4 cutoff) the risk is very high.

The method provided by the current invention to determine the risk of pathologies in women colonized by the *G. vaginalis* bacterium permits to obtain a very accurate and reliable evaluation of risk.

To obtain such determination, preferably a new method was set up for the isolation of the Gvh toxin to be used as antigen. This method consists in a particular technique, described here on, that is a further embodiment of the current invention.

The hemolytic toxin Gvh produced by the *G. vaginalis* bacterium is isolated from a suitable culture broth as, for example, the Trypto Casein Soya Broth (sold by Diagnostic Pasteur) or the Mueller Hinton Broth (sold by Oxoid).

Preferably, a broth without serum and/or plasma was set up.

In particular, this broth comprises a basic component that is Brain Heart Infusion Broth sold by Oxoid, a component of glycogen that varies from 0.5 to 10 % of the final broth volume, and a salt MgSO₄ or MgCl₂ at a concentration from 0.05 mM to 20 mM.

Preferably, glycogen varies from 0.1 al 1 %, and MgSO₄ or MgCl₂ varies from 0.2 to 10 mM, even more preferably, glycogen is 0.3 %, and MgSO₄ or MgCl₂ is 1 mM.

Surprisingly it was noted that this kind of broth without serum and/or plasma and with Magnesium and glycogen, rather than starch and glucose usually employed in well known broths, permits to obtain an optimal growth of the *G. vaginalis* bacterium and, moreover, a large increase of the Gvh toxin production.

Moreover, it was noted that the absence of detergent Tween, usually employed in the culture broth, and the absence of plasma proteins led to considerably increase the hemolytic toxin activity.

The isolation of said protein can be achieved directly from an amount of said culture broth or from the toxin eluate obtained from broth supernatant processed with normal chromatographic procedures.

This amount of broth or of toxin eluate is kept at a temperature in the range from 0 to 8 °C and then mixed with a prefixed amount of precipitating agent with chaotropic properties as, for example, methanol, ethanol, propanol, isopropanol, acetone, and ammonium sulfate.

Preferably, prechilled methanol is used at a concentration that varies from 5 to 70% of the final volume, more preferably, is 33%.

This mixture is left on ice for a time frame from 30 to 60 minutes.

Subsequently, the mixture is centrifuged at a temperature between 0 and 8°C, at 2500-5000 rpm, for a time frame from 20 to 60 minutes.

At the end of the centrifugation a pellet is obtained which consists in the toxin directly precipitated from the culture broth supernatant or from the eluate obtained through the chromatographic purification of the broth.

Subsequently, first the supernatant and then the eventually remaining methanol are eliminated from said pellet.

Finally, the pellet is recovered by suspension in deionized water or ammonium acetate solution.

If the toxin isolation is performed directly from the culture broth of *G. vaginalis*, the amount of broth drawn for the precipitation must be held in a glass or polypropilene container to avoid loss of toxin due to hydrophobic effects.

If the so obtained toxin is not used immediately for an immune enzymatic test, after centrifugation the pellet is recovered with AcNH₄, or NaCl, or NaClO₄, or (NH₄)₂SO₄, or NH₄Cl, at a concentration between 1 and 100 mM, with addition of a detergent as Tween 20 or Tween 80 or Nonidet P-40 (NP-40 sold by Calbiochem, and sold by Sigma with the name CA-630), or Brij® 35 (Calbiochem), or Lubrol®, or n-octylglucoside from 0.001 to 1%, preferably, 50mM AcNH₄, and 0.05% Tween 20. The so obtained preparation is then stored in a glass container at -80°C for long periods or at -20°C for short periods.

The hemolytic activity of the protein is maintained if the pellet is lyophilized.

If the toxin is isolated directly from the culture broth, it is better to perform a second precipitation in the same conditions described for the first precipitation.

After the second precipitation a new pellet of the Gvh toxin is obtained, this has to be recovered with deionized sterile water and conserved frozen or lyophilized at -80°C for long periods or at -20°C for short periods.

The advantage of this embodiment relies on the fact that the procedure to isolate the Gvh hemolytic toxin is easier and faster to perform because the chromatographic step is substituted with a simple precipitation.

As previously mentioned the Gvh toxin can be used to determine the anti-Gvh IgA or IgG or IgM secretory antibodies by an immune enzymatic test.

For example an ELISA test similar to that here used was described by Cauci et al. Am J Obstet Gynecol 1996; 175: 1601-5, as previously reported. It is to note that the Gvh toxin isolated with the just described method allows to perform the incubation step of the ELISA test at room temperature without being obliged to warm the incubation plates of the tested samples.

What is described till now permits to give objectively determinable markers to carefully select women who need therapy to prevent the severe pathologic complications listed before, moreover it allows to evaluate the efficacy of a performed therapy. Moreover, both the method and the isolated toxin objects of the current invention give important keys for the future preparation of vaccines effective against said pathologies and, at the same time, devoid of the large drawbacks caused by the side effects of the currently used drugs.

Here, on an indicative and not limitative example of preparation of the Gvh antigen used to perform an ELISA test in accord with the method of the current invention will be described.

### GARDNERELLA VAGINALIS BACTERIUM CULTURE.

*G. vaginalis* is grown in a culture broth without addition of serum or plasma.

The *G. vaginalis* strain can be those deposited at ATCC having the reference number 14018 or 14019 otherwise, as in the present embodiment, can be isolated from patients in which bacterial vaginosis was diagnosed.

The bacterium to be grown in the broth can be drawn from a strain conserved in glycerol, for example 200 µl of said strain could be put in a bottle containing 50 ml of broth.

Alternatively, the strain firstly grown on agar plate in a solid medium with human blood, in anaerobic conditions at 37°C, is drawn with, for example, a sterile loop to inoculate a bottle of broth from 50 to 100 ml.

After obtaining a significant growth having an A₆₂₀=0.5-1.5 OD, in which A₆₂₀ indicates absorbance at 620 nanometers, from 10 to 25 ml of culture broth are used to inoculate a new 1 liter broth bottle.

In particular, the broth sold by Oxoid with the name Brain Heart Infusion Broth (BHI) is utilized. Alternatively, Cooked Meat Medium, Mueller Hinton Broth, Liver Broth, Nutrient Broth, Schaedler Anaerobe Broth, Todd-Hewitt Broth, Tryptone Soya Broth, Tryptose Phosphate Broth, Wilkins-Chalgren Anaerobe Broth (all broth sold by Oxoid) and analogs can be used. 0.3% glycogen from oyster sold by Sigma and 1 mM MgSO₄ or MgCl₂ are added to this broth.

All inoculations are performed in an anaerobic atmosphere (inside a cabinet having a 5-10% CO₂ atmosphere, or in a jar with AnaeroGen bags sold by Oxoid) at 37°C temperature, on the average for 24 hours. If the strain was just unfrozen 48 hours may be necessary, whereas for the second broth step usually 16 hours are sufficient.

A well-grown broth has flocculent turbidity, final pH from 4.7 to 6.2 and hemolytic activity equal at least at 1HU₅₀=1 µl broth.

At the end of the bacterial growth, the broth is centrifuged at 3500 rpm for 30 minutes at 4°C and, eventually, it is filtrated through a 0.45 micron membrane for sterilization.

If the broth is not immediately processed, it is added with the detergent Tween 20 at a final concentration 0.1 % and then frozen at -20°C.

If, on the contrary, the broth has to be immediately processed, it is chilled on ice and precipitated.

### Gvh HEMOLYTIC TOXIN PRECIPITATION.

To precipitate the Gvh toxin, methanol at HPLC chromatographic grade purity is used.

Methanol is prechilled at -80°C and then an amount equal to ½ of the broth volume is added, leading to a 33% final concentration.

The broth has to be inside a glass or polypropilene container and kept on ice for about 1 hour.

After the addition of methanol to the broth, the mixture is left on ice for 30-60 minutes, then it is centrifuged for 30 minutes at 4°C and at 4000 rpm.

At the end of the centrifugation the supernatant is eliminated from the container and the methanol remaining in the pellet is evaporated. In particular, the residual methanol evaporation is performed under vacuum pomp for about 1 hour.

Later the pellet is suspended with sterile deionized water or with 50 mM AcNH₄ and 0.05 % Tween 20. This preparation has to be stored in a glass container, frozen at -80°C for long periods or at -20°C for short periods.

This first precipitation results in about 15-20 fold concentration of the starting volume of the broth (50-70 ml from 1 liter broth) and about 5 fold concentration of the hemolytic activity of the toxin.

Subsequently, a second precipitation is performed in the same conditions described above, and using only glass containers.

After the precipitation, the precipitated toxin must be suspended in sterile deionized water and stored frozen or lyophilized at -80 °C or -20 °C.

After this second precipitation, from 5 to 10 ml of toxin with hemolytic activity sufficient to make from 2500 to 5000 assays are obtained from 1 liter of broth.

### PREPARATION FOR ELISA TEST

The isolated toxin, as above described, when used for ELISA test, is diluted 1:50 in the ELISA coating buffer. The buffer consists in 0.1 M sodium carbonate at pH 9.6.

In particular, 1 ml of the isolated toxin is required to obtain 50 ml of coating solution necessary to prepare 5 plates of 96 wells, using 100 µl per well.

The ELISA test is then performed with normal procedures well known in the field. It is to note that, by the isolation method above described, such test can be carried out at room temperature.

### ELISA TEST

A flat bottom well plate is used for the ELISA assay (for example, Type I, Costar). To each well 0.100 ml of purified antigen (Gvh) in 0.1 M carbonate buffer at pH=9.6 corresponding to 100 HU₅₀ of hemolytic activity are added. The incubation is carried out at room temperature for 16 hours. Then the not well adsorbed antigen is removed through aspiration of the solution and 3 subsequent washes of the well with 0.300 ml of PBS solution containing 0.05% Tween 20 are done. To block the aspecific binding sites an incubation is performed using 0.300 ml of PBS solution containing 0.05% Tween 20 and 2% BSA for 2 hours at room temperature. In parallel an analogous blocking procedure is performed in wells not coated with the antigen that will need for the background subtraction. After aspiration of the blocking solution 0.100 ml of the biological fluid sample (eventually buffered at pH=7.2 with a 25 mM phosphate buffer) are added both to the plated coated with the antigen and to the uncoated plate (for background control). The incubation is carried out for 2 hours at room temperature. Then the biological fluid sample is removed and 3 well washes are performed as above described. 0.100 ml of buffer solution at pH 7.4 containing 1:5000 diluted F(ab')₂ anti-human IgA antibody conjugated with alkaline phosphatase (Sigma) are added. Wells are incubated for 1 hour at room temperature and then washed 3 times as above described. Then 0.100 ml of 1g/l para-nitrophenylphosphate substrate in a buffer solution made of 0.1 M glycine, 1 mM MgCl₂, 0.1 mM ZnCl₂ at pH=10.6 were added. The color developed after the hydrolysis of the substrate and release of para-nitrophenol is measured after 30-60 minute incubation at room temperature, by ELISA reader at 405 nm. Results are expressed as mOD value read in the well coated with the antigen minus the mOD value read in the not antigen coated well and incubated with the same biological fluid sample. All measurements are performed in duplicate. Two wells are incubated with a negative control (that is without anti-Gvh Ig) and two wells are incubated with two positive controls.

Several different embodiments of the just described test can be performed. In particular, para-nitrophenol can be replaced with a different substrate able to reveal the alkaline phosphatase activity, as, for example, 2-nitrophenyl phosphate that can replace 4-nitrophenyl phosphate, or a chromogenic substrate that develops an insoluble colored precipitate (necessary for the strip test). The substrate can be the combination BCIP/NBT (5-bromo-4-chloro-3-indolyl phosphate / nitro blue tetrazolium) that develops a blue/indigo color product of hydrolysis. A further substrate is the 4-methylumbelliferyl phosphate that instead gives a fluorescent product of hydrolysis that can be detected in solution or on spot.

The anti-human Ig antibody can further be conjugated with enzymes different from the alkaline phosphatase, the more common are: 1) the horseradish peroxidase, with this substrate different substrates can be employed, the most common is the tetramethylbenzidine (TMB) that, by addition of hydrogen peroxide, gives a blue color, the color development is then stopped with sulfuric acid and at the end a yellow color is read in solution at 450 nm; 2) the beta-galactosidase, also with this substrate various different substrates can be used, in particular such enzymatic activity is very useful for a strip test, because several different insoluble substrates exist that give precipitates of many different colors: the 5-bromo-4-chloro-3-indolyl galactoside (X-Gal) gives an indigo blue color, 5-bromo-6-chloro-3-indolyl galactoside gives a magenta color, the 6-chloro-3-indolyl galactoside gives a pink color, the 5-iodo-3-indolyl galactoside gives a purple color, the N-methyl-3-indolyl galactoside gives a green color and finally the 3-hydroxyindol-beta-D-galactoside gives a blue color (all the substrates listed are sold by Molecular Probes, some by Calbiochem and some by Sigma). Substrates for the beta-galactosidase that give soluble products with absorbance in the UV range to be used in ELISA as for example the 2-nitrophenyl-beta-D-galactoside (ONPG), the 4-nitrophenyl-beta-D-galactoside. Moreover there are several different substrates that give fluorescent hydrolysis products.

To obtain a signal amplification it is custom to use an anti-human Ig conjugated with biotin, the detection is then performed with avidin (or strepavidin) conjugated with a detecting enzyme (as alkaline phosphatase, peroxidase, beta-galactosidase) and the suitable chromogenic or fluorogenic substrate, that can be soluble (for the ELISA test) or insoluble (for strip, dot blot, Western blot, or gel assay).

A further object of the current invention is a kit to determine anti-Gvh IgA and/or IgG and/or IgM in a body fluid sample of women colonized by the *G. vaginalis* bacterium comprising the use of the Gvh toxin isolated accordingly to the previously described method. Preferably, the kit comprises the Gvh toxin in the amount of 2 µl equivalent to 0.02-0.04 µg protein or to 100 HU₅₀ of hemolytic activity.

As an example, the kit can comprise an ELISA plate with wells not coated with the antigen to subtract the aspecific background, a solution containing 250 mM phosphate buffer at pH=7.2 to be added to the well before incubation of the body fluid sample, a negative control of biological fluid devoid of anti-Gvh immunoglobulins, a positive control of biological fluid containing anti-Gvh immunoglobulins, a concentrated solution containing an Ig antibody conjugated with a revealing enzyme, a solution suitable to dilute the antibody concentrated solution immediately before the addition to the well, a substrate suitable for the quantitative detection of the enzymatic activity conjugated to the anti-Ig antibody, a solution suitable to dilute a concentrated solution or a tablet of substrate immediately before the addition to the well, an agent to stop the color development, a concentrated solution necessary for the wells' washings to be diluted 5 fold or 10 fold before use, an instruction leaflet with the explanations for the proper use of the assay and with the explanation of the risk of complications (absent or low (-), medium (+), high (++), very high (+++)) on the basis of the reading results.

Accordingly to a further embodiment of this invention, said kit can comprise a pH indicator and a test to determine IgA and/or IgG and/or IgM antibodies.

Preferably, the pH indicator is a pH revealing paper with a turning interval in the range between 4 and 7, whereas the antibody test is an ELISA test as that previously described.

According to a further embodiment, said kit can also comprise a sialidase and/or prolidase activity test in solution, including a vial containing a colorless substrate in solution in which to inoculate the biological sample, a container with a dispenser for the addition of the developing solution; a reference scale to evaluate the entity of the enzymatic activity by comparison with the intensity of the developed color.

According to a further embodiment, said kit can also comprise a sialidase and/or prolidase activity test in platform, comprising two membranes supported on a solid frame, and containing an enzyme substrate and an agent for the development of the color, through which the biological sample is passed; and a reference scale to evaluate the entity of the enzymatic activity by the intensity of the developed color.

According to a further embodiment, said kit can also comprise a sialidase and/or prolidase activity test on a solid support, comprising a membrane impregnated with a chromogenic or fluorogenic substrate of the enzyme and eventually an agent for the color development, supported on an inert strip, to be touched with the biological sample. Said kit will be furnished of a reference scale to evaluate the entity of the enzymatic activity by the intensity of the developed color.

In said kit, the Gvh toxin can be supported on a piece of nitrocellulose, or cellulose, or cellulose acetate, or Dacron®, or polysulfone, or PVDF, or Mylar®, that constitutes a reactive strip.

Moreover, said toxin can be supplied in a lyophilized status or frozen and available in a fashion well known in the field.

Alternatively, the kit in accord to the present invention can comprise a Quick Card well known in the field. In particular, said Quick-Card includes a membrane functionalized with the Gvh antigen, a membrane not functionalized with the Gvh antigen for the negative control, both fixed to a support and visible to the operator through an open window on the solid support. The biological fluid to be assayed is passed through the membrane so that the anti-Gvh antibodies became bonded to the test spot. After addition of a developing reagent that reacts with the anti-Gvh immunoglobulins a color is developed and is compared with a reference scale.

It is to take into consideration that the kit in accord with the invention can be favorably a combined kit. In fact, said kit can for example comprise an ELISA plate and a supported pH indicator with a sensitivity in the range from pH 4 to pH 7, or a test in solution to determine the sialidase activity and/or prolidase activity, or an ELISA plate, a pH indicator and a test on solid support to determine the sialidase and/or prolidase activity. Preferably, the solid support can be a platform as previously described, or a reactive strip routinely used for this kind or similar determinations.

Analogously, these combinations can be performed replacing the ELISA plate with for example a Quick Card.

It stands out now that, with reference to the considerations contained in the tables previously shown, and to the evaluations about the analytical test shown or available, a kit that can furnish a rapid method to determine the risk of the previously described pathologies is of major importance at the aim to give the clinician efficient tools to decide about the pharmacological treatment to administer to the patients.

As can be appreciated from what above described, the method for the determination of the risk of complications according to this invention allows to satisfy the requirements described in the introduction of the current description.

Obviously an expert operator in the field, to satisfy contingent and specific demands, could introduce several modifications and embodiments to the above described method, but all these are contained in the invention as defined by the following claims.

## Claims

1. A method for the determination of the risk of pathologies correlated with pregnancy complications and with susceptibility to sexually transmitted diseases in women colonized by *Gardnerella vaginalis* bacterium, comprising the following steps in order:
a) determination of the levels of IgA and/or IgG and/or IgM antibodies against the Gvh toxin secreted by *G. vaginalis* in a sample of body fluid;
b) provision of a value indicative of the risk;
c) comparison of said levels of IgA and/or IgG and/or IgM obtained from the step a) with said value indicative of the risk provided n step b);
d) determination of risk factor,
wherein said value indicative of the risk is obtainable by a method comprising the steps of:
i) providing a group of women colonized by *G. vaginalis*;
ii) determining the levels of anti-Gvh IgA and/or IgG and/or IgM in such a group;
iii) calculating the percentage of women of said group who had said complication or diseased and who had no complication or diseases;
iv) calculating a value and its correction with a standard factor by means of a computer statistical program on the basis of the percentage obtained in step iii) in order to evaluate the value indicative of the risk.

2. Method as set forth in claim 1, furthermore comprising after the c) step:
- determination of pH of the sample;
- comparison of the measured pH value with a prefixed value.

3. Method as set forth in claim 1, furthermore comprising after the c) step:
- determination of samples derived from women suffering BV.

4. Method as set forth in claim 1, furthermore comprising after the c) step:
- determination of the bacterial flora in said samples of vaginal fluid.

5. Method as set forth in any one of claims from 1 to 4, furthermore comprising after the b) step:
- determination of sialidase activity in said sample.

6. Method as set forth in any one of claims from 1 to 4, furthermore comprising after the c) step:
- determination of prolidase activity in said sample.

7. Method as set forth in any one of claims from 1 to 4, furthermore comprising after the c) step:
- determination of sialidase and prolidase activity in said sample.

8. Method as set forth in any one of claims from 1 to 7, in which the pathologies correlated with the colonization of the *Gardnerella vaginalis* bacterium comprise pregnancy adverse outcomes as low birth weight (LBW) or preterm delivery (PTD), preterm rupture of membranes, spontaneous abortion, infections of the amniotic fluid, post-parturn endometritis, mother-to-child transmission of HIV infection, acquisition of HIV viral infection, and of HPV viral infection, upper genital tract infections (PID), post-partum ad post-gynecologic surgery infections, endometritis, cervitis.

9. Method as set forth in any one of claims from 1 to 8, in which said value indicative of the risk is 780mOD for IgA 740 mOD for IgG 600 mOD for IgM, and the prefixed pH value is 4.7.

10. Method as set forth in any one of claims from 1 to 8, in which the prefixed IgA or IgG or IgM value is twofold the cutoff measured on healthy population and calculated from the medium value plus one standard deviation, and the prefixed pH is equal or over 4.7.

11. Method as set forth in any one of claims from 1 to 10, in which said method is performed in a body fluid sample of women from the first to the second trimester of gestation.

12. Method as set forth in any one of claims from 1 to 11, in which said method is performed in a body fluid sample of women from the seventh to the twenty-fourth week' gestation.

13. Method as set forth in any one of claims from 1 to 12, in which said method is performed in a vaginal fluid sample of women.

14. Method as set forth in claim 13, in which said method is performed in a vaginal fluid sample of pregnant women.

15. Method as set forth in any one of claims from 1 to 13, in which the population of women colonized by *G. vaginalis* is constituted by pregnant women.

16. Method as set forth in any one of claims from 1 to 13, in which the population of women colonized by *G. vaginalis* is constituted by fertile age women.

17. Method as set forth in any one of claims from 1 to 13, in which the population of women colonized by *G. vaginalis* is constituted by postmenopausal women.

18. Method as set forth in any one of claims from 1 to 17, in which the determination of the IgA and/or IgG and/or IgM levels is carried out with a suitable immune enzymatic analysis comprising the availability, as antigen, of the Gvh toxin isolated from a suitable culture broth of *G. vaginalis* by precipitation comprising the following steps:
1) to draw a predetermined amount of *G. vaginalis* culture broth or of said toxin purified by chromatography of the supernatant of *G. vaginalis* culture broth, to chill and keep said amount at a temperature from 0 to +8 °C for about 1 hour;
2) to add to said amount a determinate amount of precipitating agent at a final concentration that varies from 5 to 70 % of the final volume;
3) to keep on ice for 30-60 minutes;
4) to centrifuge at a temperature from 0 to +8 °C at 2500-5000 rpm for about 30-60 minutes in a way to obtain a supernatant and a pellet;
5) to eliminate the supernatant and then the precipitating agent remaining on the pellet;
6) to resuspend the pellet with sterile deionized water.

19. Method as set forth in claim 18, in which said precipitating agent is chosen in a group comprising methanol, ethanol, acetone, ammonium sulfate, propanol, isopropanol.

20. Method as set forth in claim 19, in which the precipitating agent is methanol at a concentration 33% of the final volume.

21. Method as set forth in any one of claims from 18 to 20, in which if the phase of IgA and/or IgG and/or IgM determination comprises the drawing of a predetermine amount of broth directly from the culture, then to the f) step a further precipitation follows repeating the step from b) to f).

22. Method as set forth in any one of claims from 18 to 21, in which at the end of the f) step the pellet is resuspended with AcNH₄ at a concentration that varies from 1 to 100 mM and a detergent at a concentration that varies from 0.01 to 1 %.

23. Method as set forth in claim 22, in which the pellet is resuspended with 50 mM AcNH₄ and 0.05 % Tween 20.

24. Method as set forth in any one of claims from 18 to 23, in which the bacterium *G. vaginalis* is grown in a broth chosen in a group consisting of Brain Hearth Infusion Broth, Cooked Meat Medium, Mueller Hinton Broth, Liver Broth, Nutrient Broth, Schaedler Anaerobe Broth, Todd-Hewitt Broth, Tryptone Soya Broth, Tryptose Phosphate Broth, Wilkins-Chalgren Anaerobe Broth, said broth being added with glycogen from 0.5 to 10 % and MgSO₄ or MgCl₂ from 0.05 to 20 mM.

25. Method as set forth in claim 24, in which the broth is BHI broth comprising 0.3 % glycogen and 1 mM MgSO₄ or MgCl₂.

26. Method as set forth in any one of claims from 18 to 25, in which the immune enzymatic analysis consists in an ELISA test

27. Method as set forth in claim 26, in which the ELISA test is performed at room temperature.

## Patentansprüche

1. Verfahren zur Bestimmung des Risikos von Pathologien in Zusammenhang mit Schwangerschaftskomplikationen und mit Empfindlichkeit auf sexuell übertragene Krankheiten bei Frauen, die eine Besiedelung mit Bakterium Gardnerella vaginalis aufweisen, umfassend die folgenden Schritte in der Reihenfolge:
a) Bestimmen der Menge an IgA- und/oder IgG- und/oder IgM-Antikörpern gegen das von G. vaginalis ausgeschiedene Gvh-Toxin in einer Probe von Körperflüssigkeit;
b) Bereitstellen eines Wertes, der das Risiko anzeigt;
c) Vergleich der Mengen an IgA und/oder IgG und/oder IgM erhalten in Schritt a) mit dem Wert, der das in Schritt b) vorgesehene Risiko anzeigt;
d) Bestimmen eines Risikofaktors,
worin der Wert, der das Risiko anzeigt, nach einem Verfahren erhältlich ist, das die Schritte umfasst:
i) Bereitstellen einer Gruppe von Frauen, die eine Besiedelung mit G. vaginalis aufweisen;
ii) Bestimmen der Mengen an anti-Gvh IgA und/oder IgG und/oder IgM in einer solchen Gruppe;
iii) Berechnen des Prozentsatzes an Frauen der Gruppe, die diese Komplikation oder diese Krankheiten aufweisen und die keine Komplikation oder Krankheiten aufweisen;
iv) Berechnen eines Wertes und seine Korrektur mit einem Standardfaktor mittels eines Computerstatistikprogramms auf Basis des in Schritt iii) erhaltenen Prozentsatzes, um den Wert zu ermitteln, der das Risiko anzeigt.

2. Verfahren nach Anspruch 1, ferner umfassend nach Schritt c):
- Bestimmen des pH der Probe;
- Vergleich des gemessenen pH-Werts mit einem festgelegten Wert.

3. Verfahren nach Anspruch 1, ferner umfassend nach Schritt c):
- Bestimmen von Proben, die von Frauen gewonnen sind, die an BV leiden.

4. Verfahren nach Anspruch 1, ferner umfassend nach Schritt c):
- Bestimmen der Bakterienflora in Vaginalfluidproben.

5. Verfahren nach einem der Ansprüche 1 bis 4, ferner umfassend nach Schritt b):
- Bestimmen der Sialidaseaktivität in der Probe.

6. Verfahren nach einem der Ansprüche 1 bis 4, ferner umfassend nach Schritt c):
- Bestimmen der Prolidaseaktivität in der Probe.

7. Verfahren nach einem der Ansprüche 1 bis 4, ferner umfassend nach Schritt c):
- Bestimmen der Sialidase- und Prolidaseaktivität in der Probe.

8. Verfahren nach einem der Ansprüche 1 bis 7, in dem die mit der Besiedelung mit Bakterium Gardnerella vaginalis in Zusammenhang stehenden Pathologien unerwünschte Wirkungen bei Schwangerschaft umfassen wie geringes Geburtsgewicht oder Frühgeburt, vorzeitiger Membransprung, Spontanabort, Infektionen des Fruchtwassers, Endometritis post partum, Übertragung einer HIV-Infektion von der Mutter aufs Kind, Erwerb einer HIV-Virusinfektion und HPV-Virusinfektion, Infektionen des oberen Genitaltrakts, Infektionen nach geburtshilflichen oder gynäkologischen Eingriffen, Endometritis, Cervitis.

9. Verfahren nach einem der Ansprüche 1 bis 8, in dem der Wert, der das Risiko anzeigt, 780 mOD für IgA, 740 mOD für IgG, 600 mOD für IgM und der festgelegte pH-Wert 4,7 beträgt.

10. Verfahren nach einem der Ansprüche 1 bis 8, in dem der festgelegte IgA- oder IgG- oder IgM-Wert zweifach der bei gesunder Population gemessene Richtwert ist und aus dem Mittelwert plus einer Standardabweichung berechnet, und der festgelegte pH gleich oder größer 4,7 ist.

11. Verfahren nach einem der Ansprüche 1 bis 10, in dem das Verfahren an einer Körperflüssigkeitsprobe von Frauen im ersten bis zweiten Trimester der Schwangerschaft durchgeführt wird.

12. Verfahren nach einem der Ansprüche 1 bis 11, in dem das Verfahren an einer Körperflüssigkeitsprobe von Frauen in der siebten bis vierundzwanzigsten Schwangerschaftswoche durchgeführt wird.

13. Verfahren nach einem der Ansprüche 1 bis 12, in dem das Verfahren an einer Vaginalfluidprobe von Frauen durchgeführt wird.

14. Verfahren nach Anspruch 13, in dem das Verfahren an einer Vaginalfluidprobe von schwangeren Frauen durchgeführt wird.

15. Verfahren nach einem der Ansprüche 1 bis 13, in dem die Population von Frauen mit Besiedelung mit G. vaginals von schwangeren Frauen gebildet ist.

16. Verfahren nach einem der Ansprüche 1 bis 13, in dem die Population von Frauen mit Besiedelung mit G. vaginals von Frauen im fruchtbaren Alter gebildet ist.

17. Verfahren nach einem der Ansprüche 1 bis 13, in dem die Population von Frauen mit Besiedelung mit G. vaginals von Frauen nach der Menopause gebildet ist.

18. Verfahren nach einem der Ansprüche 1 bis 17, in dem die Bestimmung der IgA- und/oder IgG- und/oder IgM-Mengen mit einer geeigneten Immunenzymanalyse vorgenommen wird, die die Verfügbarkeit des Gvh-Toxins als Antigen isoliert aus einer geeigneten Kulturbrühe von G. vaginalis durch Ausfällung umfasst, wobei das Verfahren die folgenden Schritte umfasst:
1) Abziehen einer bestimmten Menge an G. vaginalis Kulturbrühe oder des Toxins gereinigt durch Chromatographie des Überstands von G. vaginalis Kulturbrühe, Kühlen und Lagern der Menge bei einer Temperatur von 0 bis +8 °C für ungefähr 1 Stunde;
2) Hinzufügen einer bestimmten Menge an Ausfällmittel auf eine Endkonzentration, die von 5 bis 70 % des Endvolumens schwankt, zu dieser Menge;
3) Lagern auf Eis für 30-60 Minuten;
4) Zentrifugieren bei einer Temperatur von 0 bis +8 °C bei 2500-5000 Upm über ungefähr 30-60 Minuten, so dass ein Überstand und ein Pellet erhalten werden;
5) Eliminieren des Überstands und dann des auf dem Pellet verbliebenen Ausfällmittels;
6) erneutes Suspendieren des Pellets mit sterilem deionisiertem Wasser.

19. Verfahren nach Anspruch 18, in dem das Ausfällmittel aus einer Gruppe umfassend Methanol, Ethanol, Aceton, Ammoniumsulfat, Propanol, Isopropanol gewählt wird.

20. Verfahren nach Anspruch 19, in dem das Ausfällmittel Methanol in einer Konzentration von 33 % des Endvolumens ist.

21. Verfahren nach einem der Ansprüche 18 bis 20, in dem, wenn die Phase der IgA- und/oder IgG- und/oder IgM-Bestimmung Abziehen einer bestimmten Menge an Brühe direkt aus der Kultur umfasst, dann dem Schritt f) eine weitere Ausfällung folgt, die die Schritte von b) bis f) wiederholt.

22. Verfahren nach einem der Ansprüche 18 bis 21, in dem am Ende des Schritts f) das Pellet mit AcNH₄ bei einer Konzentration, die von 1 bis 100 mM schwankt, und einem Tensid bei einer Konzentration, die von 0,01 bis 1 % schwankt, erneut suspendiert wird.

23. Verfahren nach Anspruch 22, in dem das Pellet mit 50 mM AcNH₄ und 0,05 % Tween 20 erneut suspendiert wird.

24. Verfahren nach einem der Ansprüche 18 bis 23, in dem das Bakterium G. vaginalis in einer Brühe gezüchtet wird ausgewählt aus der Gruppe bestehend Hirn-Herz-Infusionslösung (BHI), Fleischbrühmedium, Mueller-Hinton-Brühe, Leberbrühe, Nährstoffbrühe, Schaedler anaerobe Brühe, Todd-Hewitt-Brühe, Trypton-Soja-Brühe, Tryptosephosphatbrühe, Wilkins-Chalgren anaerobe Brühe, wobei dieser Brühe Glycogen von 0,5 bis 10 % und MgSO₄ oder MgCl₂ von 0,05 bis 20 mM zugesetzt wird.

25. Verfahren nach Anspruch 24, in dem die Brühe BHI-Brühe ist umfassend 0,3 % Glycogen und 1 mM MgSO₄ oder MgCl₂.

26. Verfahren nach einem der Ansprüche 18 bis 25, in dem die Immunenzymanalyse in einem ELISA-Test besteht.

27. Verfahren nach Anspruch 26, in dem der ELISA-Test bei Raumtemperatur durchgeführt wird.

## Revendications

1. Procédé pour la détermination du risque de pathologies corrélées avec des complications de grossesse et avec une susceptibilité de maladies transmises sexuellement chez les femmes colonisées par une bactérie *Gardnerella vaginalis,* comprenant dans l'ordre les étapes suivantes :
a) détermination des niveaux d'anticorps IgA et/ou IgG et/ou IgM contre la toxine Gvh sécrétée par *G. vaginalis* dans un échantillon de fluide corporel ;
b) attribution d'une valeur indicative du risque ;
c) comparaison desdits niveaux d'IgA et/ou d'IgG et/ou d'IgM obtenus dans l'étape a) avec ladite valeur indicative du risque attribuée dans l'étape b) ;
d) détermination du facteur de risque,
dans lequel ladite valeur indicative du risque est susceptible d'être obtenue par un procédé comprenant les étapes de :
i) fournir un groupe de femmes colonisées par *G. vaginalis* ;
ii) déterminer les niveaux d'IgA et/ou d'IgG et/ou d'IgM anti-Gvh dans un tel groupe ;
iii) calculer le pourcentage de femmes dudit groupe qui avait lesdites maladies ou complication et qui n'avait pas de complication ou de maladies ;
iv) calculer une valeur et sa correction avec un facteur standard au moyen d'un programme de calcul statistique sur la base du pourcentage obtenu dans l'étape iii) de manière à évaluer la valeur indicative du risque.

2. Procédé comme énoncé dans la revendication 1, comprenant en outre après l'étape c) :
- une détermination de pH de l'échantillon ;
- une comparaison de la valeur de pH mesurée avec une valeur préfixée.

3. Procédé comme énoncé dans la revendication 1, comprenant en outre après l'étape c) :
- une détermination des échantillons dérivés des femmes souffrant de vaginose bactérienne (BV).

4. Procédé comme énoncé dans la revendication 1, comprenant en outre après l'étape c) :
- une détermination de la flore bactérienne dans lesdits échantillons de fluide vaginal.

5. Procédé comme énoncé dans l'une quelconque des revendications 1 à 4, comprenant en outre après l'étape b) :
- une détermination de l'activité de la sialidase dans ledit échantillon.

6. Procédé comme énoncé dans l'une quelconque des revendications 1 à 4, comprenant en outre après l'étape c) :
- une détermination de l'activité de la prolidase dans ledit échantillon.

7. Procédé comme énoncé dans l'une quelconque des revendications 1 à 4, comprenant en outre après l'étape c) :
- une détermination de l'activité de la sialidase et de la prolidase dans ledit échantillon.

8. Procédé comme énoncé dans l'une quelconque des revendications 1 à 7, dans lequel les pathologies corrélées avec la colonisation de la bactérie *Gardnerella vaginalis* comprennent des résultats indésirables de la grossesse comme un poids faible à la naissance (LBW) ou une naissance prématurée (PTD), une rupture prématurée des membranes, un avortement spontané, des infections du liquide amniotique, une endométrite post-partum, une transmission de la mère à l'enfant d'une infection au HIV, une acquisition d'une infection virale au HIV, et d'une infection virale au HPV, des infections génitales hautes (PID), des infections post-partum à post-opératoires gynécologiques, une endométrite, une cervite.

9. Procédé comme énoncé dans l'une quelconque des revendications 1 à 8, dans lequel ladite valeur indicative du risque est de 780 mOD pour IgA, de 740 mOD pour IgG, de 600 mOD pour IgM, et la valeur de pH préfixée est de 4,7.

10. Procédé comme énoncé dans l'une quelconque des revendications 1 à 8, dans lequel la valeur d'IgA ou d'IgG ou d'IgM préfixée est deux fois la coupe mesurée sur une population en bonne santé et calculée à partir de la valeur moyenne plus une déviation standard, et le pH préfixé est égal ou supérieur à 4,7.

11. Procédé comme énoncé dans l'une quelconque des revendications 1 à 10, dans lequel ledit procédé est effectué dans un échantillon de fluide corporel de femmes du premier au second trimestre de gestation.

12. Procédé comme énoncé dans l'une quelconque des revendications 1 à 11, dans lequel ledit procédé est effectué dans un échantillon de fluide corporel de femmes de la septième à la vingt-quatrième semaine de gestation.

13. Procédé comme énoncé dans l'une quelconque des revendications 1 à 12, dans lequel ledit procédé est effectué dans un échantillon de fluide vaginal de femme.

14. Procédé comme énoncé dans la revendication 13, dans lequel ledit procédé est effectué dans un échantillon de fluide vaginal de femme enceinte.

15. Procédé comme énoncé dans l'une quelconque des revendications 1 à 13, dans lequel la population de femmes colonisées par *G. vaginalis* est constituée de femmes enceintes.

16. Procédé comme énoncé dans l'une quelconque des revendications 1 à 13, dans lequel la population de femmes colonisées par *G. vaginalis* est constituée de femmes d'âge fertile.

17. Procédé comme énoncé dans l'une quelconque des revendications 1 à 13, dans lequel la population de femmes colonisées par *G. vaginalis* est constituée de femmes post-ménopausiques.

18. Procédé comme énoncé dans l'une quelconque des revendications 1 à 17, dans lequel la détermination des niveaux d'IgA et/ou d'IgG et/ou d'IgM est effectuée avec une analyse immuno-enzymatique appropriée comprenant la disponibilité, en tant qu'antigène, de la toxine Gvh isolée à partir d'un bouillon de culture approprié de *G. vaginalis* par précipitation comprenant les étapes suivantes :
1) de retirer une quantité prédéterminée de bouillon de culture de *G. vaginalis* ou de ladite toxine purifiée par chromatographie du surnageant du bouillon de culture de *G. vaginalis*, de refroidir et de garder ladite quantité à une température de 0 à +8 °C pendant environ 1 heure ;
2) d'ajouter à ladite quantité une quantité déterminée d'agent précipitant à une concentration finale qui varie de 5 à 70 % du volume final ;
3) de garder sur de la glace pendant 30 à 60 minutes ;
4) de centrifuger à une température de 0 à +8 °C à 2500-5000 rpm pendant environ 30-60 minutes de manière à obtenir un surnageant et une boulette ;
5) d'éliminer le surnageant et ensuite l'agent précipitant restant sur la boulette ;
6) de remettre en suspension la boulette avec de l'eau déminéralisée stérile.

19. Procédé comme énoncé dans la revendication 18, dans lequel ledit agent précipitant est choisi dans un groupe comprenant le méthanol, l'éthanol, l'acétone, le sulfate d'ammonium, le propanol, l'isopropanol.

20. Procédé comme énoncé dans la revendication 19, dans lequel ledit agent précipitant est le méthanol à une concentration de 33 % du volume final.

21. Procédé comme énoncé dans l'une quelconque des revendications 18 à 20, dans lequel si la phase de détermination d'IgA et/ou d'IgG et/ou d'IgM comprend l'enlèvement d'une quantité prédéterminée de bouillon directement de la culture, alors jusqu'à l'étape f) une précipitation supplémentaire suit répétant l'étape de b) à f).

22. Procédé comme énoncé dans l'une quelconque des revendications 18 à 21, dans lequel à la fin de l'étape f) la boulette est remise en suspension avec AcNH₄ à une concentration qui varie de 1 à 100 mM et un détergent à une concentration qui varie de 0,01 à 1 %.

23. Procédé comme énoncé dans la revendication 22, dans lequel la boulette est remise en suspension avec 50 mM d'AcNH₄ et 0,05 % de Tween 20.

24. Procédé comme énoncé dans l'une quelconque des revendications 18 à 23, dans lequel la bactérie G. vaginalis est mise en croissance dans un bouillon choisi dans un groupe constitué par un bouillon d'infusion coeur-cervelle (BHI), un milieu de viande cuite (CMM), un bouillon de Mueller-Hinton, un bouillon de foie, un bouillon nutritif, un bouillon anaérobie de Schaedler, un bouillon de Todd-Hewitt, un bouillon de tryptone-soja, un bouillon de tryptose-phosphate, un bouillon anaérobie de Wilkins-Chalgren, ledit bouillon étant ajouté avec du glycogène de 0,5 à 10 % et du MgSO₄ ou du MgCl₂ de 0,05 à 20 mM.

25. Procédé comme énoncé dans la revendication 24, dans lequel le bouillon est un bouillon BHI comprenant 0,3 % de glycogène et 1 mM de MgSO₄ ou MgCl₂.

26. Procédé comme énoncé dans l'une quelconque des revendications 18 à 25, dans lequel l'analyse immuno-enzymatique consiste en un test ELISA.

27. Procédé comme énoncé dans la revendication 26, dans lequel le test ELISA est effectué à température ambiante.
